# EUROPEAN PATENT SPECIFICATION

(11) **EP 2 679 571 B1**
(45) Date of publication and mention of the grant of the patent: **13.12.2017**
(21) Application number: 12382264.5
(22) Date of filing: 29.06.2012
(51) Int. Cl.: C07C 68/06, C07C 68/08, C07C 69/96, B01D 3/14, B01D 3/00

(54) **Method and apparatus for the production of diaryl carbonate**
Verfahren und Vorrichtung zur Herstellung von Diarylcarbonat
Procédé et appareil pour la production de carbonate de diaryle

(43) Date of publication of application: 01.01.2014
(73) Proprietor: SABIC Global Technologies B.V., 4612 PX Bergen op Zoom (NL)
(72) Inventor: Vic Fernandez, Ignacio, 30390 Murcia (ES); Ferrer Nadal, Sergio, 30390 Murcia (ES); Caballero, Jose Antonio, 03540 Alicante (ES); Javaloyes Antón, Juan, 03201 Alicante (ES)
(74) Representative: Carpintero Lopez, Francisco

(56) References cited:
- US-A1- 2004 266 974

## Description

### BACKGROUND

The present disclosure relates generally to a method and an apparatus for the production of diaryl carbonate, and especially to a method and an apparatus for the production of diphenyl carbonate ("DPC").

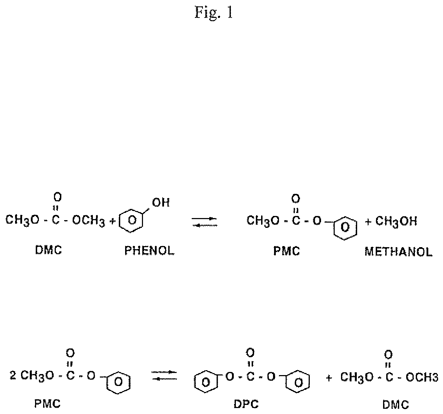

Diaryl carbonate, such as DPC, is an important reactant in the production of polycarbonates. For example, polycarbonates can be manufactured by reacting Bisphenol A with DPC. Polycarbonates are useful materials valued for their physical and optical properties. As the uses for polycarbonates have increased, the efficient production of diaryl carbonates has become of greater significance. Early processes for the production of diaryl carbonates utilized phosgene as a reactant. The toxicity of phosgene, however, prompted the development of a non-phosgene process. As shown schematically in FIG. 1, the non-phosgene process involves a two-step process. First, a dialkyl carbonate, such as dimethyl carbonate ("DMC") reacts with an aromatic alcohol, such as phenol, to produce an alkyl aryl carbonate (e.g., phenyl methyl carbonate ("PMC")) and an alkyl alcohol (alkanol), such as methanol, in the presence of a transesterification catalyst. Then, in the second step, two molecules of the alkyl aryl carbonate undergo a disproportionation reaction to produce one molecule of diaryl carbonate, such as DPC and one molecule of dialkyl carbonate, such as DMC. The reaction shown in FIG. 1 is the known desired reaction, but as also is known there are a number of side reactions that occur, producing unwanted by-products. These by-products can interfere with continuing production of the desired product, reduce the efficiency of the overall process, and in some cases produce waste streams requiring special handling for disposal. For example, this is the case with respect to alkyl aryl ethers, such as anisole, which is a side product produced by the reaction of dialkyl carbonate and aromatic alcohol. Other side-reactions, such as the rearrangement of aryl alkyl carbonates or aryl aryl carbonates, might produce other by-products of organics having high boiling points. Thus, a challenge is the development of a method that can minimize the quantities and effects of the reaction by-products, while providing a good yield of the desired product, specifically DPC.

U.S. Patent 6,294,684 discloses a method and an apparatus for the production of diaryl carbonate, such as DPC, using a series of distillation columns. Despite these advances, however, there is a continued need for methods and apparatuses exhibiting reduced energy consumption and requiring lower investment costs by having less processing equipment, specifically fewer distillation columns, while also capable of producing purified diaryl carbonate, especially purified DPC, with minimized reaction by-products.

### BRIEF DESCRIPTION

Disclosed in various embodiments are methods and apparatuses for the production of diaryl carbonate, especially DPC.

In an embodiment, a method for production of diaryl carbonate, can comprise: introducing reactants comprising dialkyl carbonate, an aromatic alcohol, and a transesterification catalyst to a first reactive distillation column (C21) to produce alkyl aryl carbonate and alkyl alcohol; recovering from the first reactive distillation column (C21) a first top stream (stream 5) comprising dialkyl carbonate and alkyl alcohol, a first bottom stream (stream 6) comprising alkyl aryl carbonate, aromatic alcohol, dialkyl carbonate, aryl alkyl ether, and transesterification catalyst, and a first side stream (stream 13) comprising dialkyl carbonate, aryl alkyl ether, and alkyl alcohol; introducing the first bottom stream (stream 6) into a second reactive distillation column (C32) without passing through another reactive distillation column to produce diaryl carbonate by disproportionation of the alkyl aryl carbonate; recovering from the second reactive distillation column (C32) a second bottom stream (stream 15) comprising diaryl carbonate and alkyl aryl carbonate, and a second top stream (stream 16) comprising aromatic alcohol, alkyl aryl ether, and dialkyl carbonate, and optionally recycling the second top stream (stream 16) to the first reactive distillation column (C21); introducing the first side stream (stream 13) to a first rectification column (C81); recovering from the first rectification column (C81) a third bottom stream (stream 12) comprising alkyl aryl ether and a third top stream (stream 14) comprising dialkyl carbonate and alkyl alcohol, and optionally recycling the third top stream (stream 14) to the first reactive distillation column (C21); introducing the first top stream (stream 5) into a second rectification column (C41); and recovering from the second rectification column (C41) a fourth top stream (stream 7) comprising dialkyl carbonate /alkyl alcohol azeotrope and a fourth bottom stream (stream 8) comprising dialkyl carbonate, and optionally recycling the fourth bottom stream (stream 8) to the first reactive distillation column (C21);
introducing the second bottom stream (stream 15) to a flash vessel (V51); recovering from the flash vessel (V51) a fifth bottom stream (stream 10) comprising catalyst and diaryl carbonate, and a fifth top stream (stream 9) comprising diaryl carbonate, alkyl aryl carbonate and aromatic alcohol; introducing the fifth bottom stream (stream 10) to an evaporator (C52); and recovering from the evaporator (C52) a sixth top stream (stream 11) comprising diaryl carbonate and a sixth bottom stream (stream 20) comprising the catalyst and diaryl carbonate, and recycling the catalyst to first reactive distillation column (C21);
wherein the method does not comprise an additional reactive distillation column other than the first reactive distillation column (C21) and the second reactive distillation column (C32).

In an embodiment, an apparatus for continuous production of diaryl carbonate by reaction of a dialkyl carbonate and an aromatic alcohol in the presence of a transesterification catalyst can comprise: a first reactive distillation column (C21), a second reactive distillation column (C32), a first rectification column (C81), a second rectification column (C41), and a plurality of lines for transporting reactant and product streams. The first reactive distillation column (C21) can be connected to input lines for introduction of reactants, and to a first transfer line, a second transfer line and a third transfer line, the first transfer line extends from the first reactive distillation column (C21) to an inlet of the second rectification column (C41), the second transfer line extends from the first reactive distillation column (C21) to an inlet of the second reactive distillation column (C32) without passing through another reactive distillation column, and the third transfer line extends from the side of the first reactive distillation column (C21) to an inlet of the first rectification column (C81). The second reactive distillation column (C32) can optionally be connected to a first recycle line, the first recycle line extends from the second reactive distillation column (C32) to an inlet of the first reactive distillation column (C21), and a fourth transfer line, the fourth transfer line extends from the second reactive distillation column (C32) for providing diaryl carbonate. The first rectification column (C81) can be connected to a fifth transfer line, the fifth transfer line extends from the first rectification column (C81) to an inlet of the first reactive distillation column (C21), and a first product line extends from the first rectification column (C81) providing alkyl aryl ether. The second rectification column (C41) can be connected to a second product line for providing dialkyl carbonate/alkyl alcohol azeotrope, and optionally a second recycle line extends from the second rectification column (C41) to the first reactive distillation column (C21);
further comprising a flash vessel (V51), an evaporator (C52), and a first purification column (C61), wherein the flash vessel (V51) is connected to a sixth line and a seventh line, the sixth line extends from the top of the flash vessel (V51) to an inlet of the first purification column (C61), and the seventh line extends from the bottom of the flash vessel (V51) to an inlet of the evaporator (C52);
and wherein the apparatus does not comprise an additional reactive distillation column other than the first reactive distillation column (C21) and the second reactive distillation column (C32).

The above described and other features are exemplified by the following figures and detailed description.

### BRIEF DESCRIPTION OF THE DRAWINGS

Refer now to the Figures, which are exemplary and non-limiting embodiments, and wherein the like elements are numbered alike.
FIG. 1 schematically depicts the two-step reaction of dimethyl carbonate and phenol (PhOH) to produce diphenyl carbonate.
FIG. 2 schematically depicts a plant design for the production of diaryl carbonate, such as DPC.
FIG. 3A schematically depicts direct exchange heat integration between the condenser and reboiler of FIG. 2.
FIG. 3B schematically depicts a heat carrier between the condenser and reboiler of FIG. 2.

### DETAILED DESCRIPTION

The disclosure herein is directed to how to improve the overall efficiency, reduce energy consumption, and reduce the overall cost associated with the production of diaryl carbonate, such as DPC. Due to the present process and system, the number of sequential distillation columns needed to produce and purify DPC can be reduced. Herein, processing equipment is redesigned and processing parameters such as, for example, pressure and temperature are adjusted. Surprising reductions in energy consumption during DPC production can be achieved, as evidenced by the below-described examples.

A more complete understanding of the components, processes, and apparatuses disclosed herein can be obtained by reference to the accompanying drawings. These figures (also referred to herein as "FIG.") are merely schematic representations based on convenience and the ease of demonstrating the present disclosure, and are, therefore, not intended to indicate relative size and dimensions of the devices or components thereof and/or to define or limit the scope of the exemplary embodiments. Although specific terms are used in the following description for the sake of clarity, these terms are intended to refer only to the particular structure of the embodiments selected for illustration in the drawings, and are not intended to define or limit the scope of the disclosure. In the drawings and the following description below, it is to be understood that like numeric designations refer to components of like function.

The principal reaction in embodiments disclosed herein is the afore-described reaction set forth in FIG. 1. It is noted that this reaction can be carried out using various dialkyl carbonates and various aromatic alcohols, such as those known in the art and disclosed, for example, in U.S. Patent Nos. 5,210,268, 5,344,954, 5,705,673, and 6,294,684. Examples of dialkyl carbonates are those that include a carbonate group disposed between two alkyl groups. Specific examples include, DMC, diethyl carbonate, dipropyl carbonate, dibutyl carbonate, and dihexyl carbonate. An example of an aromatic alcohol is phenol, and further specific examples of aromatics include unsubstituted phenol o-, m- or p-cresol, o-, m- or p-chlorophenol, o-, m- or p-methoxyphenol, 2,6-dimethylphenol, 2,4-dimethylphenol, 3,4-dimethylphenol, 1-napthol and 2-naphthol.

Typical reactants useful on an industrial scale are DMC and phenol, which react to produce DPC. It is noted that these specific reactants will be primarily referred to below in the description for ease of reference and merely as non-limiting and exemplary materials. It should be understood that this usage is merely for ease of reference and that no limitation on embodiments disclosed herein to only the specific use of these materials is intended.

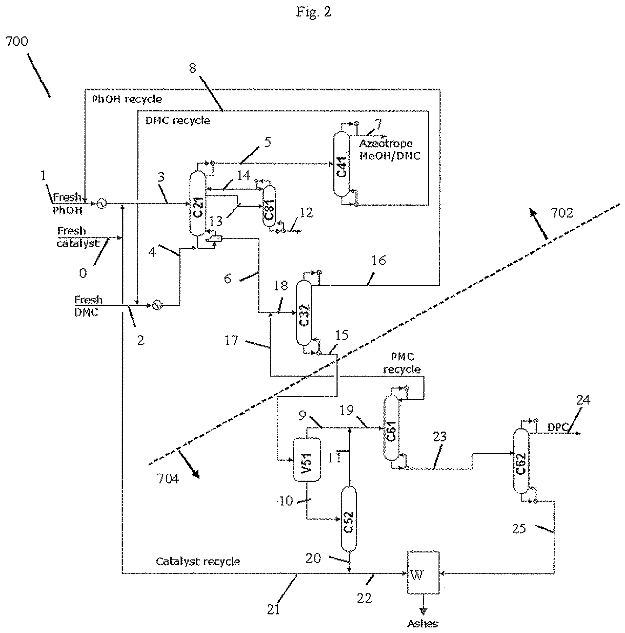

FIG. 2 schematically depicts a diaryl carbonate production plant/recovery apparatus/design 700, in accordance with embodiments disclosed herein, comprising a reaction/distillation section 702 and a purification section 704. The apparatus 700 comprises four columns, C21, C81, C32, and C41 in the reaction/distillation section 702 of FIG. 2. Previously, five or more columns typically were required in this section of a DPC production plant facility.

Various feed, product, and recycle streams indicated by the numbers 1-25 also are shown in FIG. 2. It is further noted that it will be appreciated by persons skilled in the art that the positioning of the various streams/lines as described herein as being, e.g., in the "top", "middle", "bottom", or "side" of a particular column is relative because the actual position at which material is to be introduced or recovered is dependent on the conditions being maintained in the particular column. For example, a stream entering the "bottom" of a column may actually enter several stages above the sump including the reboiler of the column, and a line/stream exiting the "top" of the column may actually exit several stages below the top stage including the condenser of the column. Thus, such terms herein are included for ease of reference to describe a general orientation regarding various columns and lines/streams and such terms are not meant to be limiting to one exact location. Also, although for illustrative purposes, the Figures and their description may depict singular vessels, such as reaction vessels or mixing vessels, it is understood that multiple vessels in series or parallel may be used where suitable.

With further reference to FIG. 2, Columns C21 and C32 can be reactive distillation columns. Thus, these columns can each have a rectification section and/or a reaction section in which a chemical reaction occurs. In general, the reactive portion of the column can be furnished with packings or fixed internals to provide at least one reactive distillation stage. For example, the reaction section of column C21 can provide greater than or equal to 5, such as 5 to 60, reactive stages, specifically greater than or equal to 10, such as 10 to 40, distillation stages. Known dumped packings and/or arranged packings can be employed. Specifically, packings having a large surface area, good wetting and residence time of the liquid phase, such as, for example, Novolax rings, CY packings, can be used. Fixed internals, such as tray columns also can be employed, and specific examples include sieve trays, valve trays, and bubble-cap trays.

Column C41 and column C81 can be rectification columns. These columns can carry out a separation of materials based upon boiling point, without driving a concurrent chemical reaction. The afore-referenced columns can be interconnected by a series of feed/recycle lines, which serve to transport streams comprising reactants and/or products. The direction of flow for each line is indicated in FIG. 2. Various valves, heaters, and other fittings, optionally, can be included with the feed/recycle lines shown in FIG. 2 in adapting the design to a particular installation.

The apparatus depicted in FIG. 2 can be used in accordance with a method to produce diaryl carbonate, specifcally DPC, according to embodiments disclosed herein. With further reference to FIG. 2, Stream 0 can comprise a transesterification catalyst and phenol. Catalysts include titanium compounds, such as, titanium tetraphenoxide, titanium isopropylate, titanium tetrachloride, organotin compounds, and compounds of copper, lead, zinc, iron, and zirconium, and combinations comprising at least one of the foregoing. Stream 1 can comprise fresh phenol (PhOH). Stream 2 can comprise fresh dialkyl carbonate, specifically DMC. Starting materials/reactants can be introduced to column C21 through streams 3 and 4. Stream 3 is a combination of stream 1, recycle stream 16, and recycle stream 21, discussed in further detail below, which can comprise phenol, catalyst, and some DMC. Stream 21 comprises a recycle catalyst. Stream 4 is a mixture of stream 2 and recycle stream 8, comprising mainly DMC with some minor amounts of phenol and side-reaction products recovered from, for example, the bottom of the second rectification column C41.

Stream 4 can be fed to, for example, the bottom of column C21, specifically to the reboiler. Stream 4 can be a liquid or a vapor, depending upon the type of reboiler used. For example, if an external reboiler, e.g., a kettle reboiler, is used, stream 4 can enter column C21 as a vapor. Stream 3 can be fed as a liquid into, e.g., the middle section of column C21, more specifically at a location at or near the top of the reactive distillation section. The feed rate of streams 3 and 4 can be such that the molar ratio of dialkyl carbonate to aromatic alcohol, which is introduced into column C21, is between 0.1 and 10, specifically between 0.5 and 5, and more specifically between 1 and 3. Dialkyl carbonate can be provided in excess through stream 4 because the dialkyl carbonate can serve as both a reactant and as a stripping agent, which facilitates removal of alkyl alcohol (alkanol) produced in the transesterification reaction. This removal can increase the rate of production of alkyl aryl carbonate in column C21.

A transesterification reaction can be carried out in column C21 at, for example, a temperature greater than or equal to 100°C, specifically greater than or equal to 130°C, and more specifically greater than or equal to 140°C. Examples of temperature ranges for carrying out the reaction include, 100°C to 300°C, specifically 110°C to 270°C, and more specifically 120°C to 250°C. The operating pressure at the top of column C21 can be greater than or equal to 0.5 barg (50 kPa), specifically greater than or equal to 2 barg (200 kPa), and more specifically greater than or equal to 3 barg (300 kPa). Reactant products and unreacted starting materials can be removed from column C21 in a continuous manner through streams 5 and 6. Stream 5, which can be drawn from the top of column C21, can comprise unreacted dialkyl carbonate and also can comprise the alkyl alcohol produced in the transesterification reaction. Stream 5 can pass to second rectification column C41 for processing and recovery. Rectification column C41 can produce a by-product stream 7 comprising an azeotropic mixture of dialkyl carbonate and essentially all of the alkyl alcohol produced in the process. Stream 7 can be condensed and reused as a feed stream for a complementary dialkyl carbonate production process, e.g. DMC production process, without further purification.

Column C41 also can be furnished with packings or fixed internals to provide, for example, greater than or equal to 3, specifically greater than or equal to 5, more specifically between 5 to 50, distillation stages. For example, known dumped packings and/or arranged packings can be employed.

The temperature profile of column C41 can be greater than or equal to 10°C, specifically greater than or equal to 50°C. Examples of temperature ranges for the temperature profile in the column C41 are 10°C to 200°C, specifically 50°C to 110°C. The operating pressure in column C41 can be greater than or equal to 0.1 bar (10 kPa), specifically greater than or equal to 0.5 bar (50 kPa). Examples of pressure ranges include 0.1 to 10 bar (1000 kPa), specifically 0.5 to 3 bar (50 to 300 kPa).

Stream 6, which can be drawn from, e.g., the bottom of column C21, such as from the reboiler of column C21, can comprise the alkyl aryl carbonate produced in column C21, in combination with unreacted starting materials, alkyl aryl ether and catalyst. Stream 6 can be augmented by the addition of a recycle stream (stream 17) comprising alkyl aryl carbonate and aromatic alcohol to form stream 18. Stream 17 can be an alkyl aryl carbonate-containing stream recovered from the purification section 704 during optional purification of diaryl carbonate in purification section 704, which is described in further detail below. Such augmentation can result in an improvement in the overall production of diaryl carbonate. Thus, stream 6, now augmented with stream 17 to form stream 18 and comprising alkyl aryl carbonate, can, according to some embodiments, enter the second reactive distillation column C32, without passing through another reactive distillation column, to produce diaryl carbonate by disproportionation of the alkyl aryl carbonate. Column C32 can be operated to further drive the reaction toward the desired diaryl carbonate product, while separating other materials for recycle. Two streams can be removed from column C32. The first is a product stream 15, which can be removed from the bottom of column C32, and can comprise essentially all of the diaryl carbonate produced together with residual catalyst, some alkyl aryl carbonate and unwanted high boiling by-products. The product stream 15 can optionally be further distilled and purified in purification section 704 if additional purification is desired.

Stream 16, which can be removed from the top of column C32 as a recycle stream, can comprise essentially all of the unreacted aromatic alcohol starting material, and some dialkyl carbonate and by produced alkyl aryl ether, and be recycled to make up part of stream 3.

Column C32 can be operated at a temperature greater than or equal to 90°C, specifically greater than or equal to 100°C, and more specifically greater than or equal to 110°C. Examples of temperature ranges include 100°C to 140°C, specifically 120°C to 250°C, and 110°C to 240°C. The operating pressure in column C32 can be greater than or equal to 10 mbar (1 kPa), specifically greater than or equal to 50 mbar (5 kPa), and more specifically greater than or equal to 100 mbar (10 kPa). Examples of pressure ranges include, 50 mbar to 3 bar (5 to 300 kPa), 50 mbar to 1 bar (5 to 100 kPa), and 200 mbar to 900 mbar (20 to 90 kPa).

In some exemplary embodiments disclosed herein, recovering an additional stream (stream 13) from first reactive distillation column C21, e.g., such as a side draw, which can then be fed into first rectification column C81, can help to reduce the overall number of distillation columns needed to effectively produce diaryl carbonate. For example, stream 13 can comprise dialkyl carbonate, alkanol and aryl alkyl ether, and can be fed directly into column C81. A product stream 14 can be recovered from, e.g., the top of C81 and recycled back to C21. For example, stream 14 can comprise mainly dialkyl carbonate with some alkanol. An aryl alkyl ether rich stream can be recovered from, e.g., the bottom of column C81 through stream 12, which can be stored in, e.g. a storage tank for further purification or sold as a product. Removal of aryl alkyl ether from the process in this fashion can help avoid its build up.

Column C81 can be operated at a temperature greater than or equal to 50°C, specifically greater than or equal to 80°C, more specifically greater than or equal to 100°C. Examples of temperature ranges include 100°C to 190°C. The operating pressure in column C81 can be greater than or equal to 0.5 bar (50 kPa), specifically greater than or equal to 0.7 bar (70 kPa), and more specifically greater than or equal to 1 bar (100 kPa). Examples of a pressure range include 1 to 4 bars (100 to 400 kPa).

Optionally, stream 15 described above can be sent to flash vessel V51 in which diaryl carbonate, alkyl aryl carbonate and phenol can be vaporized and separated from the catalyst and by produced high boilers (HBs) which are organics having a boiling point greater than or equal to diphenyl carbonate (excluding catalyst), such as phenyl-2-methoxy benzonates, xanthone, etc. Streams 9 and 10 can then be recovered from flash vessel V51. Stream 10 can be recovered from, e.g., the bottom of flash vessel V51 and can comprise catalyst, high boilers and diaryl carbonate, which can be introduced to evaporator C52. Stream 9, which can comprise diaryl carbonate, alkyl aryl carbonate, and aromatic alcohol can be recovered from top of flash vessel V51 and mixed with stream 11 to form stream 19 and enter column C61 located in purification section 704 of FIG. 2. Flash vessel V51 can be operated at a temperature greater than or equal to 140°C, specifically greater or equal to 150°C, more specifically greater or equal to 160°C. The operating pressure in flash vessel V51 can be greater than or equal to 1 mbar (100 Pa), specifically greater than or equal to 10 mbar (1 kPa), and more specifically greater than or equal to 15 mbar (1.5 kPa). Examples of pressure ranges include 15 to 150 mbar (1.5 to 15 kPa).

Stream 11 can be recovered from, e.g., the top of evaporator C52 and comprise diaryl carbonate to be mixed with stream 9. Stream 20 can be recovered from, e.g., the bottom of column C52 and split into recycle stream 21 to recycle catalyst back to column C21 by mixing with stream 1, and stream 22 comprising waste products to enter waste station W for processing and disposing the residue. Evaporator C52 can be operated at a temperature greater than or equal to 150°C, specifically greater or equal to 160°C, more specifically greater or equal to 170°C. The operating pressure in evaporator C52 can be greater than or equal to 1 mbar (100 Pa), specifically greater than or equal to 2 mbar (200 Pa), and more specifically greater than or equal to 5 mbar (500 Pa). Examples of pressure ranges include 5 to 50 mbar (500 Pa to 5 kPa).

Two streams, streams 17 and 23, can be recovered from first purification column C61, which operates as a distillation column. Stream 17, described above, which can comprise unreacted alkyl aryl carbonate and aromatic alcohol, can be recovered from, e.g., the top of column C61 and recycled to help form stream 18, which enters the second reactive distillation column C32. The temperature profile of column C61 can be greater than or equal to 30°C, specifically greater than or equal to 60°C. Examples of temperature ranges for the temperature profile in the column C61 are 30°C to 140°C, specifically 60°C to 190°C. The operating pressure in column C61 can be greater than or equal to 1 mbar (100 Pa), specifically greater than or equal to 5 mbar (500 Pa). Examples of pressure ranges are 10 to 70 mbar (1 to 7 kPa).

Stream 23 can be recovered from, e.g., the bottom of first purification column C61 and can comprise diaryl carbonate. Stream 23 can optionally enter second purification column C62 for further purification and recovery of a diaryl carbonate product stream 24, which can comprise, e.g., pure DPC (impurities in trace amounts). From, e.g., the bottom of second purification column C62, stream 25 can be obtained, which can comprise mainly waste products sent to waste station W for processing and disposal. The temperature profile of column C62 can be greater than or equal to 50°C, specifically greater than or equal to 100°C. Examples of temperature ranges for the temperature profile in the column C62 include 140°C to 180°C, specifically 150°C to 190°C. The operating pressure in column C62 can be greater than or equal to 1 mbar (100 Pa), specifically greater than or equal to 5 mbar (500 Pa). Examples of pressure ranges are 5 to 70 mbar (500 Pa to 7 kPa).

In some exemplary embodiments such as the afore-described configuration of FIG. 2, the conditions in column C21, such as temperature, pressure and molar ratio of dialkyl carbonate and aromatic alcohol feeds can be selected and optimized to reduce or otherwise control the concentration of, e.g., DMC in the bottom product stream 6. By means of an exemplary strategy, an additional reactive distillation column, such as column B1 described in the afore-referenced U.S. Patent 6,294,684, can be avoided because, for example, most of the DPC formation can advantageously take place just in the second reactive distillation column C32 described herein. Column C32 can be directly fed by, e.g., the bottom product stream of column C21 (stream 6) together with a PMC recycle stream (stream 17) from purification section 704 (e.g., column C61 overheads) to form stream 18. Thus, it is not necessary to have an additional column between the flow from a first reactive distillation column C21 and a second reactive distillation column C32. By way of contrast, U.S. Patent 6,294,884 discloses an additional reactive distillation column B1.

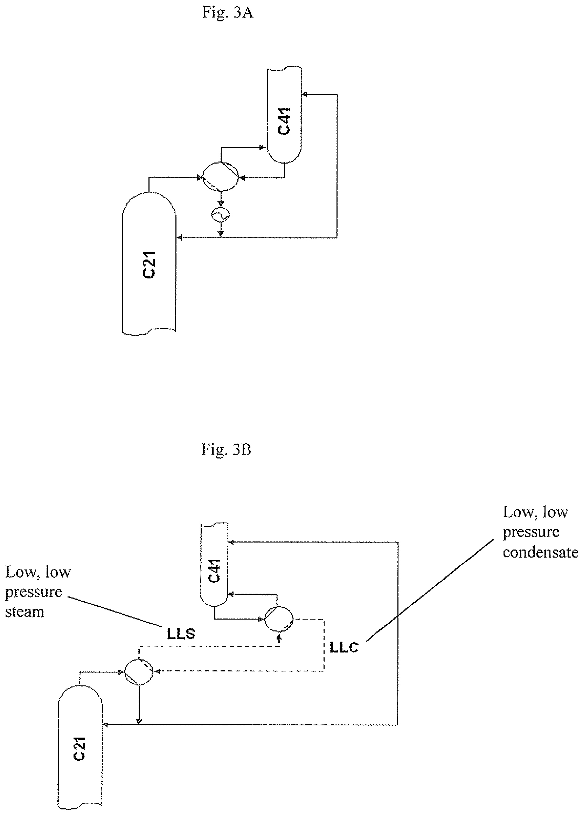

As a result of significant effort by the inventors, certain processing equipment, such as the afore-referenced additional distillation column B1, can be eliminated, and/or column C41 need not be coupled to an additional reactive distillation column (see, e.g., the coupling of columns B2 and B1 of U.S. Patent 6,294,684, which is not required by embodiments herein). Thus, column C41 herein can exhibit an enhanced flexibility that can result in other advantages. For example, the operating pressures of column C41 can be conveniently adjusted to thermally integrate, directly or indirectly, by using a heat carrier as low pressure steam (LLS), the condenser duty released from column C21 to fulfill the reboiler duty of column C41. More specifically, as shown in FIG. 3A, the reboiler of column C41 can also be the condenser of column C21 and vice versa. Therefore, the heating medium for the reboiler of column C41 can be the condensing vapors from column C21. Although the same piece of equipment could be used for both the condenser of C21 and the reboiler of C41, an additional heat exchanger can be employed because the heat required to condense the vapors from C21 is generally greater than the heat required from the reboiler of C41. As also shown in FIG. 3B, the condenser duty from C21 can be used to generate low-pressure steam (LLS) from low pressure water condensate (LLC) that, at the same time, can be used as the heating medium for the reboiler of column C41. In another exemplary embodiment, the independent operation of column C41 allows the proportion of, e.g., methanol in the methanol/DMC azeotrope, to be tailored. Different azeotrope compositions can be obtained by increasing the pressure profile along the C41 column. A DMC-rich stream can be recovered and recycled to feed column C21 from, e.g., the bottom of column C41. In some exemplary embodiments, decoupling C41 from an additional reactive distillation column allows for, e.g., the amount of DMC in the methanol/DMC azeotrope stream (stream 7), to be tailored.

In an embodiment, a method for production of diaryl carbonate, can comprise: introducing reactants comprising dialkyl carbonate, an aromatic alcohol, and a transesterification catalyst to a first reactive distillation column (C21) to produce alkyl aryl carbonate and alkyl alcohol; recovering from the first reactive distillation column (C21) a first top stream (stream 5) comprising dialkyl carbonate and alkyl alcohol, a first bottom stream (stream 6) comprising alkyl aryl carbonate, aromatic alcohol, dialkyl carbonate, aryl alkyl ether, and transesterification catalyst, and a first side stream (stream 13) comprising dialkyl carbonate, aryl alkyl ether, and alkyl alcohol; introducing the first bottom stream (stream 6) into a second reactive distillation column (C32) without passing through another reactive distillation column to produce diaryl carbonate by disproportionation of the alkyl aryl carbonate; recovering from the second reactive distillation column (C32) a second bottom stream (stream 15) comprising diaryl carbonate and alkyl aryl carbonate, and a second top stream (stream 16) comprising aromatic alcohol, alkyl aryl ether, and dialkyl carbonate, and optionally recycling the second top stream (stream 16) to the first reactive distillation column (C21); introducing the first side stream (stream 13) to a first rectification column (C81); recovering from the first rectification column (C81) a third bottom stream (stream 12) comprising alkyl aryl ether and a third top stream (stream 14) comprising dialkyl carbonate and alkyl alcohol, and optionally recycling the third top stream (stream 14) to the first reactive distillation column (C21); introducing the first top stream (stream 5) into a second rectification column (C41); and recovering from the second rectification column (C41) a fourth top stream (stream 7) comprising dialkyl carbonate /alkyl alcohol azeotrope and a fourth bottom stream (stream 8) comprising dialkyl carbonate, and optionally recycling the fourth bottom stream (stream 8) to the first reactive distillation column (C21).

In the various embodiments of the method: (i) the method can further comprise introducing the second bottom stream (stream 15) to a flash vessel (V51), recovering from the flash vessel (V51) a fifth bottom stream (stream 10) comprising catalyst and diaryl carbonate, and a fifth top stream (stream 9) comprising diaryl carbonate, alkyl aryl carbonate and aromatic alcohol, introducing the fifth bottom stream (stream 10) to an evaporator (C52), and recovering from the evaporator (C52) a sixth top stream (stream 11) comprising diaryl carbonate and a sixth bottom stream (stream 20) comprising the catalyst and diaryl carbonate, and optionally recycling the catalyst to first reactive distillation column (C21); and/or (ii) the method can further comprise introducing the sixth top stream (stream 11) to a first purification distillation column (C61), recovering from the first purification column (C61) a seventh bottom stream (stream 23) comprising diaryl carbonate and a seventh top stream (stream 17) comprising unreacted aryl alkyl carbonate and alkyl alcohol, and optionally recycling the seventh top stream (stream 17) to the second reactive distillation column (C32), introducing the seventh bottom stream (stream 23) to a second purification column (C62), and recovering from the second purification column (C62) an eighth top stream (stream 24) comprising diaryl carbonate and an eighth bottom stream (stream 25) comprising waste products; and/or (iii) the first reactive distillation column (C21) can be maintained at a temperature of 120°C to 270°C, and a pressure at the top of the first reactive distillation column (C21) of 4 to 6 bars (400 to 600 kPa); and/or (iv) the second reactive distillation column (C32) can be maintained at a temperature of 140°C to 240°C, and a pressure at the top of the second reactive distillation column (C32) of 0.2 to 0.9 bars (20 to 90 kPa); and/or (iv) the first rectification column (C81) can be maintained at a temperature of 100°C to 190°C, and a pressure at the top of the column (C81) of 1 to 3 bars (100 to 300 kPa); and/or (v) the second rectification column (C41) can be maintained of 50°C to 110°C, and a pressure at the top of the column (C41) of 0.5 to 2 bars (50 to 200 kPa); and/or (vi) the first purification column (C61) can be maintained at a temperature of 60°C to 190°C, and a pressure at the top of the column (C61) of 10 to 70 mbar (1 to 7 kPa); and/or (vii) the catalyst can comprise titanium tetraphenoxide; and/or (viii) the first rectification column (C41) can comprise a reboiler, and wherein heat of condensation from the first reactive distillation column (C21) supplies heat to a reboiler of the second rectification column (C41); and/or (ix) the dialkyl carbonate can comprise dimethyl carbonate; and/or (x) the diaryl carbonate comprises diphenyl carbonate; and/or (xi) the aromatic alcohol comprises phenol; and/or (xii) the alkyl aryl carbonate comprises methyl phenyl carbonate; and/or (xiii) the aryl alkyl ether comprises methyl phenyl ether; and/or (xiv) the alkyl alcohol comprises methanol; and/or (xv) the dialkyl carbonate is dimethyl carbonate, the diaryl carbonate is diphenyl carbonate, the aromatic alcohol is phenol, the alkyl aryl carbonate is methyl phenyl carbonate, the aryl alkyl ether is methyl phenyl ether, and the alkyl alcohol is methanol; and/or (xvi) the catalyst comprises a titanium compound, an organotin compound, or a combination comprising at least one of the foregoing catalysts; and/or (xvii) the catalyst comprises titanium tetraphenoxide, titanium isopropylate, titanium tetrachloride, or a combination comprising at least one of the foregoing catalysts.

In an embodiment, an apparatus for continuous production of diaryl carbonate by reaction of a dialkyl carbonate and an aromatic alcohol in the presence of a transesterification catalyst, can comprise: a first reactive distillation column (C21), a second reactive distillation column (C32), a first rectification column (C81), a second rectification column (C41), and a plurality of lines for transporting reactant and product streams. The first reactive distillation column (C21) can be connected to input lines for introduction of reactants, and to a first transfer line, a second transfer line and a third transfer line, the first transfer line extends from the first reactive distillation column (C21) (e.g., the top thereof) to an inlet of the second rectification column (C41), the second transfer line extends from the bottom of first reactive distillation column (C21) to an inlet of the second reactive distillation column (C32) without passing through another reactive distillation column, and the third transfer line extends from the side of the first reactive distillation column (C21) to an inlet of the first rectification column (C81). The second reactive distillation column (C32) can optionally be connected to a first recycle line, the first recycle line extends from the second reactive distillation column (C32) (e.g., the top thereof) to an inlet of the first reactive distillation column (C21), and a fourth transfer line, the fourth transfer line extends from the bottom of the second reactive distillation column (C32) for providing diaryl carbonate. The first rectification column (C81) can be connected to a fifth transfer line, the fifth transfer line extends from the first rectification column (C81) (e.g., the top thereof) to an inlet of the first reactive distillation column (C21), and a first product line extends from the first rectification column (C81) providing alkyl aryl ether (e.g., extends from the bottom of the first rectification column (C81)). The second rectification column (C41) can be connected to a second product line for providing dialkyl carbonate/ alkyl alcohol azeotrope from the second rectification column (C41) (e.g., from the top thereof), and optionally a second recycle line extends from the second rectification column (C41) (e.g., the bottom thereof) to the first reactive distillation column (C21) (e.g., the bottom thereof).

In the various embodiments of the apparatus: (i) the apparatus can further comprise a flash vessel (V51), an evaporator (C52), and a first purification column (C61), wherein the flash vessel (V51) is connected to a sixth line and a seventh line, the sixth line extends from the flash vessel (V51) (e.g., the top thereof) to an inlet of the first purification column (C61), and the seventh line extends from the bottom of the flash vessel (V51) to an inlet of the evaporator (C52); and/or (ii) the evaporator (C52) can optionally be connected to an eighth line and a third recycle line, the eighth line extends from the evaporator (C52) (e.g., the top thereof) to an inlet of the first purification column (C61), and the third recycle line extends from the bottom of the evaporator (C52) to an inlet of the first reactive distillation column (C21); and/or (iii) the apparatus can further comprise a second purification column (C62), wherein the first purification column (C61) is optionally connected to a fourth recycle line and a ninth line, the fourth recycle line extends from the first purification column (C61) (e.g., the top thereof) to an inlet of the second reactive distillation column (C32), and the ninth line extends from the bottom of the first purification column (C61) to an inlet of the second purification column (C62); and/or (iv) the second purification column (C62) is connected to a third product line and a fourth product line, the third product line extends from the second purification column (C62) (e.g., the top thereof) for providing diaryl carbonate, and the fourth product line extends from the bottom of the second purification column (C62) providing a waste stream; and/or (v) the second rectification column (C41) comprises a reboiler, and wherein heat of condensation from the first reactive distillation column (C21) supplies heat to the reboiler of the second rectification column (C41); and/or (vi) the dialkyl carbonate can comprise dimethyl carbonate; and/or (vii) the diaryl carbonate comprises diphenyl carbonate; and/or (viii) the aromatic alcohol comprises phenol; and/or (ix) the alkyl aryl carbonate comprises methyl phenyl carbonate; and/or (x) the aryl alkyl ether comprises methyl phenyl ether; and/or (xi) the alkyl alcohol comprises methanol; and/or (xii) the dialkyl carbonate is dimethyl carbonate, the diaryl carbonate is diphenyl carbonate, the aromatic alcohol is phenol, the alkyl aryl carbonate is methyl phenyl carbonate, the aryl alkyl ether is methyl phenyl ether, and the alkyl alcohol is methanol; and/or (xiii) the catalyst comprises a titanium compound, an organotin compound, or a combination comprising at least one of the foregoing catalysts; and/or (xiv) the catalyst comprises titanium tetraphenoxide, titanium isopropylate, titanium tetrachloride, or a combination comprising at least one of the foregoing catalysts.

The process and apparatus described above, in accordance with embodiments, are further illustrated by the following non-limiting Example. It is noted that the Example below is based, in part, on computer simulation for a production facility as shown, e.g., in FIG. 2.

### EXAMPLE

In this example and with reference to FIG. 2 described above, 13,939 kilograms/hour (kg/h) of fresh phenol (PhOH) (stream 1) was mixed with a 38,342 kg/h phenolic recycle stream (stream 16) (72.8 weight percent (wt%) phenol (PhOH), 26.9 wt% dimethyl carbonate, 0.01 wt% methanol (MeOH), and 0.01 wt% anisole) and preheated to 147°C.

Next, 2,031 kg/h of recycled catalyst (stream 21) comprising 66.8 wt% titanium tetraphenoxide catalyst, 20.0 wt% diphenyl carbonate and 13.2 wt% of high boiling compounds recovered from the purification section 704 of the process, was merged with the phenol streams 1 and 16 and fed at a middle height to the first reactive distillation column (C21). In order to compensate for catalyst losses from the purge, 176 kg/h of fresh catalyst (stream 0) (comprising 25 wt% of titanium tetraphenoxide catalyst and 75 wt% phenol) was continuously fed to the catalyst recycle loop. Additionally, 8,794kg/h of pure DMC (stream 2) was mixed with 37,884 kg/h of a recycled stream of DMC (stream 8, being substantially pure DMC (greater than 99 wt% purity)), and directly fed to the reboiler of the first reactive distillation column C21.

The first reactive distillation column C21 included greater than or equal to twenty distillation trays in the rectifying section and greater than or equal to twenty-four reactive trays with a hold-up of 0.7 cubic meters (m³) in the stripping section. A kettle-type reboiler with a hold-up of 5m³ supplied the heating of the column. The first reactive distillation column C21 also was equipped with a condenser. The temperature and pressure profiles were, respectively, 241°C and 5.1 bars (510 kPa) in the reboiler, and 127°C and 4.7 bars (470 kPa) at the top of the column.

The distillate rate (stream 5) was 44,730 kg/h, comprising 89.2 wt% DMC and 10.7 wt% methanol, while the reflux ratio of the first reactive distillation column C21 was 1.55. The bottom rate (stream 6) was 56,297 kg/h with a composition of 49.6 wt% phenol, 32.6 wt% PMC, 8.7 wt% DMC, 6.0 wt% DPC, 2.5 wt% catalyst, 0.5 wt% high boiling compounds , 0.06 wt% methanol and 0.04 wt% anisole.

A side draw (stream 13) of 4,000 kg/h (91.3 wt% DMC, 7.1 wt% anisole, 1.5 wt% MeOH, 0.07 wt% PhOH) was taken from the liquid phase of the seventh stage of the rectifying section (counting from top). This lateral draw stream was fed to the top stage of first rectification column C81, which included greater than or equal to nine stripping stages, a condenser and a reboiler. An anisole rich stream (stream 12) of 138 kg/h comprising 98.9 wt% anisole, 1.0 wt% phenol and 0.1 wt% DMC was obtained as a bottom product from column C81 and removed from the process, while 3,862 kg/h comprising 94.6 wt% DMC, 3.8 wt% anisole and 1.6 wt% methanol, was obtained as distillate (stream 14) from column C81 and recycled back to the seventh stage of the first reactive distillation column C21.

The distillate product (stream 5) from the first reactive distillation column C21 was fed to the fifteenth stage of the second rectification column C41, which included greater than or equal to twenty-eight stages, a condenser, and a reboiler. The second rectification column C41 produced a DMC-rich stream (stream 8) that was recycled back to the reboiler of the first reactive distillation column C21. The first rectification column C41 produced 6,847 kg/h of a stream (stream 7) having a composition near the azeotrope methanol/DMC (70.0 wt% methanol, 29.2 wt% DMC, 0.8 wt% CO₂) at a pressure of 1.39 bars (139 kPa) at the top of the column using a reflux ratio of 3.6. The bottom product stream 6 from the first reactive distillation column C21 and 4,240 kg/h of recycled PMC (stream 17) (comprising 92.3 wt% PMC, 3.8 wt% phenol, 2.7 wt% DPC and 1.2 wt% DMC) were fed to the second reactive distillation column C32 in whose bottom section a PMC disproportionation reaction took place. A phenol-rich top stream (stream 16) was obtained from second reactive distillation column C32 and recycled to the first reactive distillation column C21 while stream 15 comprising 56,297 kg/h comprising 49.6 wt% PhOH, 32.6 wt% PMC, 8.7 wt% DMC, 2.5 wt% catalyst and 0.5 wt% high boiling point compounds was sent to a flash vessel V51 and the rest of the purification section of the plant, to finally obtain 15,764 kg/h of pure DPC with impurities in trace amounts (stream 24) as the outlet of this purification section (i.e., less than 0.01 wt% impurities based upon the total weight of the pure DPC).

In addition to the reduction of piping and process equipment, embodiments disclosed herein can save, for example, greater than or equal to 10%, specifically, greater than or equal to 15%, and even greater than or equal to 19% of the operating energy consumption compared to other designs that produce the same quantity of DPC. For example, Table 1 below shows a detailed comparison of raw material consumptions, anisole generation and energy consumed for a DPC production of 15 tons per hour (ton/h) of DPC with use of a comparative process (as described in, for example, U.S. Patent No. 7,141,641 B2), in comparison to embodiments disclosed herein.

| Table 1 | | |
|---|---|---|
| | Comparative process | Example 1 |
| Fresh phenol consumption, kg/h | 13,384 | 13,391 |
| Fresh DMC consumption, kg/h | 8,345 | 8,372 |
| DPC production, kg/h | 15,000 | 15,000 |
| Anisole production, kg/h | 115 | 134 |
| HHS (high, high pressure) steam consumption, ton/h | 76.57 | 62.06 |
| % Energy savings | - | 18.9 |

In general, embodiments may alternately comprise (e.g., include), consist of, or consist essentially of, any appropriate components herein disclosed. The embodiments may additionally, or alternatively, be formulated so as to be devoid, or substantially free, of any components, materials, ingredients, adjuvants or species used in the prior art compositions or that are otherwise not necessary to the achievement of the function and/or objectives of the embodiments.

As used herein, a trace amount is an amount of less than 0.01 wt% based upon a total weight of the product. All ranges disclosed herein are inclusive of the endpoints, and the endpoints are independently combinable with each other (e.g., ranges of "up to 25 wt%, or, more specifically, 5 wt% to 20 wt%", is inclusive of the endpoints and all intermediate values of the ranges of "5 wt% to 25 wt%," etc.). "Combination" is inclusive of blends, mixtures, alloys, reaction products, and the like. Furthermore, the terms "first," "second," and the like, herein do not denote any order, quantity, or importance, but rather are used to distinguish one element from another. The terms "a" and "an" and "the" herein do not denote a limitation of quantity, and are to be construed to cover both the singular and the plural, unless otherwise indicated herein or clearly contradicted by context. The suffix "(s)" as used herein is intended to include both the singular and the plural of the term that it modifies, thereby including one or more of that term (e.g., the film(s) includes one or more films). Reference throughout the specification to "one embodiment", "another embodiment", "an embodiment", and so forth, means that a particular element (e.g., feature, structure, and/or characteristic) described in connection with the embodiment is included in at least one embodiment described herein, and may or may not be present in other embodiments. In addition, it is to be understood that the described elements may be combined in any suitable manner in the various embodiments.

## Claims

1. A method for production of diaryl carbonate, comprising:
introducing reactants comprising dialkyl carbonate, an aromatic alcohol, and a transesterification catalyst to a first reactive distillation column (C21) to produce alkyl aryl carbonate and alkyl alcohol;
recovering from the first reactive distillation column (C21) a first top stream (stream 5) comprising dialkyl carbonate and alkyl alcohol, a first bottom stream (stream 6) comprising alkyl aryl carbonate, aromatic alcohol, dialkyl carbonate, aryl alkyl ether, and transesterification catalyst, and a first side stream (stream 13) comprising dialkyl carbonate, aryl alkyl ether, and alkyl alcohol;
introducing the first bottom stream (stream 6) into a second reactive distillation column (C32) without passing through another reactive distillation column to produce diaryl carbonate by disproportionation of the alkyl aryl carbonate;
recovering from the second reactive distillation column (C32) a second bottom stream (stream 15) comprising diaryl carbonate and alkyl aryl carbonate, and a second top stream (stream 16) comprising aromatic alcohol, alkyl aryl ether, and dialkyl carbonate, and optionally recycling the second top stream (stream 16) to the first reactive distillation column (C21);
introducing the first side stream (stream 13) to a first rectification column (C81);
recovering from the first rectification column (C81) a third bottom stream (stream 12) comprising alkyl aryl ether and a third top stream (stream 14) comprising dialkyl carbonate and alkyl alcohol, and optionally recycling the third top stream (stream 14) to the first reactive distillation column (C21);
introducing the first top stream (stream 5) into a second rectification column (C41); and
recovering from the second rectification column (C41) a fourth top stream (stream 7) comprising dialkyl carbonate /alkyl alcohol azeotrope and a fourth bottom stream (stream 8) comprising dialkyl carbonate, and optionally recycling the fourth bottom stream (stream 8) to the first reactive distillation column (C21);
introducing the second bottom stream (stream 15) to a flash vessel (V51);
recovering from the flash vessel (V51) a fifth bottom stream (stream 10) comprising catalyst and diaryl carbonate, and a fifth top stream (stream 9) comprising diaryl carbonate, alkyl aryl carbonate and aromatic alcohol;
introducing the fifth bottom stream (stream 10) to an evaporator (C52); and
recovering from the evaporator (C52) a sixth top stream (stream 11) comprising diaryl carbonate and a sixth bottom stream (stream 20) comprising the catalyst and diaryl carbonate, and recycling the catalyst to first reactive distillation column (C21);
wherein the method does not comprise an additional reactive distillation column other than the first reactive distillation column (C21) and the second reactive distillation column (C32).

2. The method of Claim 1, further comprising:
introducing the sixth top stream (stream 11) to a first purification distillation column (C61);
recovering from the first purification column (C61) a seventh bottom stream (stream 23) comprising diaryl carbonate and a seventh top stream (stream 17) comprising unreacted aryl alkyl carbonate and alkyl alcohol, and recycling the seventh top stream (stream 17) to the second reactive distillation column (C32);
introducing the seventh bottom stream (stream 23) to a second purification column (C62); and
recovering from the second purification column (C62) an eighth top stream (stream 24) comprising diaryl carbonate and an eighth bottom stream (stream 25) comprising waste products.

3. The method of any of Claims 1-2, wherein the first reactive distillation column (C21) is maintained at a temperature of 120°C to 270°C, and a pressure at the top of the first reactive distillation column (C21) of 4 to 6 bars (400 to 600 kPa).

4. The method of any of Claims 1-5, wherein the second reactive distillation column (C32) is maintained at a temperature of 140°C to 240°C, and a pressure at the top of the second reactive distillation column (C32) of 0.2 to 0.9 bars (20 to 90 kPa).

5. The method of any of Claims 1-4, wherein the first rectification column (C81) is maintained at a temperature of 100°C to 190°C, and a pressure at the top of the column (C81) of 1 to 3 bars (100 to 300 kPa).

6. The method of any of Claims 1-5, wherein the second rectification column (C41) is maintained of 50°C to 110°C, and a pressure at the top of the column (C41) of 0.5 to 2 bars (50 to 200 kPa).

7. The method of any of Claims 1-6, wherein the first purification column (C61) is maintained at a temperature of 60°C to 190°C, and a pressure at the top of the column (C61) of 10 to 70 mbar (1 to 7 kPa).

8. The method of any of Claims 1-7, wherein the catalyst comprises a titanium compound, an organotin compounds, or a combination comprising at least one of the foregoing catalysts.

9. The method of any of Claims 1-8, wherein the catalyst comprises titanium tetraphenoxide, titanium isopropylate, titanium tetrachloride, or a combination comprising at least one of the foregoing catalysts.

10. The method of any of Claims 1-9, wherein the catalyst comprises titanium tetraphenoxide.

11. The method of any of Claims 1-10, wherein the first rectification column (C41) comprises a reboiler, and wherein heat of condensation from the first reactive distillation column (C21) supplies heat to a reboiler of the second rectification column (C41).

12. The method of any of Claims 1-11, wherein the dialkyl carbonate comprises dimethyl carbonate, the diaryl carbonate comprises diphenyl carbonate, the aromatic alcohol comprises phenol, the alkyl aryl carbonate comprises methyl phenyl carbonate, the aryl alkyl ether comprises methyl phenyl ether, and the alkyl alcohol comprises methanol.

13. An apparatus for continuous production of diaryl carbonate by reaction of a dialkyl carbonate and an aromatic alcohol in the presence of a transesterification catalyst, comprising:
a first reactive distillation column (C21), a second reactive distillation column (C32), a first rectification column (C81), a second rectification column (C41), and a plurality of lines for transporting reactant and product streams;
wherein the first reactive distillation column (C21) is connected to input lines for introduction of reactants, and to a first transfer line, a second transfer line and a third transfer line, the first transfer line extends from the top of the first reactive distillation column (C21) to an inlet of the second rectification column (C41), the second transfer line extends from the bottom of first reactive distillation column (C21) to an inlet of the second reactive distillation column (C32) without passing through another reactive distillation column, and the third transfer line extends from the side of the first reactive distillation column (C21) to an inlet of the first rectification column (C81);
wherein the second reactive distillation column (C32) is optionally connected to a first recycle line, the first recycle line extends from the top of the second reactive distillation column (C32) to an inlet of the first reactive distillation column (C21), and a fourth transfer line, the fourth transfer line extends from the bottom of the second reactive distillation column (C32) for providing diaryl carbonate;
wherein the first rectification column (C81) is connected to a fifth transfer line, the fifth transfer line extends from the top of the first rectification column (C81) to an inlet of the first reactive distillation column (C21), and a first product line extends from the bottom of the first rectification column (C81) providing alkyl aryl ether; and
wherein the second rectification column (C41) is connected to a second product line for providing dialkyl carbonate/ alkyl alcohol azeotrope from the top of the second rectification column (C41), and optionally a second recycle line extends from the bottom of the second rectification column (C41) to the bottom of the first reactive distillation column (C21)
further comprising a flash vessel (V51), an evaporator (C52), and a first purification column (C61), wherein the flash vessel (V51) is connected to a sixth line and a seventh line, the sixth line extends from the top of the flash vessel (V51) to an inlet of the first purification column (C61), and the seventh line extends from the bottom of the flash vessel (V51) to an inlet of the evaporator (C52); and
wherein the apparatus does not comprise an additional reactive distillation column other than the first reactive distillation column (C21) and the second reactive distillation column (C32).

14. The apparatus of Claim 13, wherein the evaporator (C52) is connected to an eighth line and a third recycle line, the eighth line extends from the top of the evaporator (C52) to an inlet of the first purification column (C61), and the third recycle line extends from the bottom of the evaporator (C52) to an inlet of the first reactive distillation column (C21).

15. The apparatus of any of Claims 12-14, further comprising a second purification column (C62), wherein the first purification column (C61) is connected to a fourth recycle line and a ninth line, the fourth recycle line extends from the top of the first purification column (C61) to an inlet of the second reactive distillation column (C32), and the ninth line extends from the bottom of the first purification column (C61) to an inlet of the second purification column (C62).

16. The apparatus of Claim 15, wherein the second purification column (C62) is connected to a third product line and a fourth product line, the third product line extends from the top of the second purification column (C62) for providing diaryl carbonate, and the fourth product line extends from the bottom of the second purification column (C62) providing a waste stream.

17. The apparatus of any of Claims 12-16, wherein the second rectification column (C41) comprises a reboiler, and wherein heat of condensation from the first reactive distillation column (C21) supplies heat to the reboiler of the second rectification column (C41).

18. The apparatus of any of Claims 12-17, wherein the dialkyl carbonate comprises dimethyl carbonate, the diaryl carbonate comprises diphenyl carbonate, the aromatic alcohol comprises phenol, the alkyl aryl carbonate comprises methyl phenyl carbonate, the alkyl aryl ether comprises methyl phenyl ether and the alkyl alcohol comprises methanol.

## Patentansprüche

1. Verfahren zur Herstellung eines Diarylcarbonats, umfassend:
Einleiten von Reaktanten, umfassend Dialkylcarbonat, einen aromatischen Alkohol und Transesterifizeirungskatalysatoren in eine erste reaktive Destillationssäule (C21) zum Herstellen von Alkylarylcarbonat und Alkylalkohol;
Rückgewinnen, aus der ersten reaktiven Destillationssäule (C21) eines ersten oberen Stromes (Strom 5), der Dialkylcarbonat und Alkylalkohol umfasst, eines ersten unteren Stroms (Strom 6), umfassend Alkylarylcarbonat, aromatischen Alkohol, Dialkylcarbonat, Arylaklylether und Transestefirizierungskatalysator, und einen ersten Seitenstrom (Strom 13), umfassend Dialkylcarbonat, Arylalkylether und Alkylalkohol;
Einleiten des ersten unteren Stroms (Strom 6) in eine zweite reaktive Destillationssäule (C32) ohne Durchlaufen einer weiteren reaktiven Destillationssäule zum Erzeugen von Diarylcarbonat durch Disproportionierung des Alkylarylcarbonats;
Rückgewinnen, von der zweiten reaktiven Destillationssäule (C32), eines zweiten unteren Stroms (Strom 15), umfassend Diarylcarbonat und Alkylarylcarbonat, und eines zweiten oberen Stroms (Strom 16), umfassend aromatischen Alkohol, Alkylarylether und Dialkylcarbonat, und wahlweise Rückführen des zweiten oberen Stroms (Strom 16) zur ersten reaktiven Destillationssäule (C21);
Einleiten des ersten Seitenstroms (Strom 13) in eine erste Rektifiziersäule (C81);
Rückgewinnen, aus der ersten Rektifiziersäule (C81), eines dritten unteren Stroms (Strom 12), umfassend Alkylarylether und eines dritten oberen Stroms (Strom 14), umfassend Dialkylcarbonat und Alkylalkohol, und wahlweise Rückführen des dritten oberen Stroms (Strom 14) in die erste reaktive Destillationssäule (C21);
Einleiten des ersten oberen Stroms (Strom 5) in eine zweite Rektifiziersäule (C41); und
Rückgewinnen, aus der zweiten Rektifiziersäule (C41), eines vierten oberen Stroms (Strom 7), umfassend Dialkylcarbonat/Alkylalkoholazeotrop, und eines vierten unteren Stroms (Strom 8), umfassend Dialkylcarbonat, und wahlweise Rückführen des vierten unteren Stroms (Strom 8) in die erste reaktive Destillationssäule (C21);
Einleiten des zweiten unteren Stroms (Strom 15) in einen Flash-Behälter (V51);
Rückgewinnen, aus dem Flash-Behälter (V51) eines fünften unteren Stroms (Strom 10), umfassend einen Katalysator und Diarylcarbonat und eines fünften oberen Stroms (Strom 9), umfassend Diarylcarbonat, Alkylarylcarbonat und aromatischen Alkohol;
Einleiten des fünften unteren Stroms (Strom 10) in einen Verdampfer (C52); und
Rückgewinnen, aus dem Verdampfer (C52) eines sechsten oberen Stroms (Strom 11), umfassend Diarylcarbonat und einen sechsten unteren Strom (Strom 20), umfassend den Katalysator und Diarylcarbonat, und Wiederherstellen des Katalysators für die erste reaktive Destillationssäule (C21);
wobei das Verfahren keine zusätzliche reaktive Destillationssäule umfasst, die von de ersten reaktiven Destillationssäule (C21) und der zweiten reaktiven Destillationssäule (C32) verschieden ist.

2. Verfahren nach Anspruch 1, ferner umfassend:
Einleiten des sechsten oberen Stroms (Strom 11) zu einer ersten Reinigungsdestillationssäule (C61);
Rückgewinnen, von der ersten Reinigungssäule (C61) eines siebten unteren Stroms (Strom 23), umfassend Diarylcarbonat und einen siebten oberen Strom (Strom 17), umfassend nicht umgesetztes Arylalkylcarbonat und Alkylalkohol, und Wiederherstellen des siebten oberen Stroms (Strom 17) für die zweite reaktive Destillationssäule (C32);
Einleiten des siebten unteren Stroms (Strom 23) zu einer zweiten Reinigungssäule (C62); und
Rückgewinnen, von der zweiten Reinigungssäule (C62), eines achten oberen Stroms (Strom 24), umfassend Diarylcarbonat und einen achten unteren Strom (Strom 25), umfassend Abfallprodukte.

3. Verfahren nach einem der vorhergehenden Ansprüche 1 bis 2, wobei die erste reaktive Destillationssäule (C21) bei einer Temperatur von 120°C bis 270°C gehalten wird, und einem Druck an der Oberseite der ersten reaktiven Destillationssäule (C21) von 4 bis 6 bar (400 bis 600 kPa).

4. Verfahren nach einem der Ansprüche 1 bis 5, wobei die zweite reaktive Destillationssäule (C32) bei einer Temperatur von 140°C bis 240°C gehalten wird, und einem Druck an der Oberseite der zweiten reaktiven Destillationssäule (C32) von 0,2 bis 0,9 bar (20 bis 90 kPa).

5. Verfahren nach einem der Ansprüche 1 bis 4, wobei die erste Rektifiziersäule (C81) bei einer Temperatur von 100°C bis 190°C und einem Druck an der Oberseite der Säule (C81) von 1 bis 3 bar (100 bis 300 kPa) gehalten wird.

6. Verfahren nach einem der Ansprüche 1 bis 5, wobei die zweite Rektifiziersäule (C41) bei einer Temperatur von 50°C bis 110°C und einem Druck an der Oberseite der Säule (C41) von 0,5 bis 2 bar (50 bis 200 kPa) gehalten wird.

7. Verfahren nach einem der Ansprüche 1 bis 6, wobei die erste Reinigungssäule (C61) bei einer Temperatur von 60°C bis 190°C und einem Druck an der Oberseite der Säule (C61) von 10 bis 70 mbar (1 bis 7 kPa) gehalten wird.

8. Verfahren nach einem der Ansprüche 1 bis 7, wobei der Katalysator eine Titanverbindung, Organotinverbindungen oder eine Kombination umfasst, die mindestens einen der vorstehenden Katalysatoren umfasst.

9. Verfahren nach einem der Ansprüche 1 bis 8, wobei der Katalysator Titantetraphenoxid, Titanisopropylat, Titantetrachlorid oder eine Kombination umfasst, die mindestens einen der vorstehenden Katalysatoren umfasst.

10. Verfahren nach einem der Ansprüche 1 bis 9, wobei der Katalysator Titantetraphenoxid umfasst.

11. Verfahren nach einem der Ansprüche 1 bis 10, wobei die erste Rektifiziersäule (C41) einen Aufkocher umfasst und wobei die Wärme der Kondensation von der ersten reaktiven Destillationssäule (C21) Wärme an einen Aufkocher der zweiten Rektifiziersäule (C41) abgibt.

12. Verfahren nach einem der Ansprüche 1 bis 11, wobei das Dialkylcarbonat Dimethylcarbonat umfasst, das Diarylcarbonat Diphenylcarbonat umfasst, der aromatische Alkohol Phenol umfasst, das Alkylarylcarbonat Methylphenylcarbonat umfasst, der Arylalkylether Methylphenylether umfasst und der Alkylalkohol Methanol umfasst.

13. Vorrichtung zur durchgehenden Herstellung von Diarylcarbonat durch Umsetzung eines Dialkylcarbonats und eines aromatischen Alkohols in Gegenwart eines Transesterifizierungskatalysators, umfassend:
eine erste reaktive Destillationssäule (C21), eine zweite reaktive Destillationssäule (C32), eine erste Rektifiziersäule (C81), eine zweite Rektifiziersäule (C41) und eine Mehrzahl von Leitungen zum Transportieren von Reaktant- und Produktströmen;
wobei die erste reaktive Destillationssäule (C21) mit Eingangsleitungen zur Einleitung von Reaktanten verbunden ist, und mit einer ersten Transferleitung, einer zweiten Transferleitung und einer dritten Transferleitung, wobei sich die erste Transferleitung von der Oberseite der ersten reaktiven Destillationssäule (C21) zu einem Einlass der zweiten Rektifziersäule (C41) erstreckt, sich die zweite Transferleitung von der Unterseite der ersten reaktiven Destillationssäule (C21) zu einem Einlass der zweiten reaktiven Destillationssäule (C32) erstreckt, ohne eine weitere reaktive Destillationsäule zu durchlaufen und sich die dritte Transferleitung von der Seite der ersten reaktiven Destillationssäule (C21) zu einem Einlass der ersten Rektifiziersäule (C81) erstreckt;
wobei die zweite reaktive Destillationssäule (C32) wahlweise mit einer ersten Rückführleitung verbunden ist, sich die erste Rückführleitung von der Oberseite der zweiten reaktiven Destillationssäule (C32) zu einem Einlass der ersten reaktiven Destillationssäule (C21) erstreckt, und eine vierte Transferleitung, wobei sich die vierte Transferleitung von der Unterseite der zweiten reaktiven Destillationssäule (C32) zum Bereitstellen von Diarylcarbonat erstreckt;
wobei die erste Rektifiziersäule (C81) mit einer fünften Transferleitung verbunden ist, sich die fünfte Transferleitung von der Oberseite der Rektifiziersäule (C81) zu einem Einlass der ersten reaktiven Destillationssäule (C21) erstreckt, und sich eine erste Produktleitung von der Unterseite der ersten Rektifiziersäule (C81) erstreckt, die Alkylarylether bereitstellt; und
wobei die zweite Rektifiziersäule (C41) mit einer zweiten Produktleitung zum Bereitstellen von Dialkylcarbonat/Alkylalkoholazeotrop von der Oberseite der zweiten Rektifiziersäule (C41) verbunden ist, und sich wahlweise eine zweite Rückführleitung von der Unterseite der zweiten Rektifiziersäule (C41) zur Unterseite der ersten reaktiven Destillationssäule (C21) erstreckt,
ferner umfassend einen Flash-Behälter (V51), einen Verdampfer (C52) und eine erste Reinigungssäule (C61), wobei der Flash-Behälter (V51) mit einer sechsten Leitung und einer siebten Leitung verbunden ist, wobei sich die sechste Leitung von der Oberseite des Flash-Behälters (V51) zu einem Einlass der ersten Reinigungssäule (C61) erstreckt und sich die siebte Leitung von der Unterseite des Flash-Behälters (V51) zu einem Einlass des Verdampfers (C52) erstreckt; und
wobei die Vorrichtung keine zusätzliche reaktive Destillationssäule umfasst, die von der ersten reaktiven Destillationssäule (C21) und der zweiten reaktiven Destillationssäule (C32) verschieden ist.

14. Vorrichtung nach Anspruch 13, wobei der Verdampfer (C52) mit einer achten Leitung und einer dritten Rückführleitung verbunden ist, wobei sich die achte Leitung von der Oberseite des Verdampfers (C52) zu einem Einlass der ersten Reinigungssäule (C61) erstreckt und sich die dritte Rückführleitung von der Unterseite des Verdampfers (C52) zu einem Einlass der ersten reaktiven Destillationssäule (C21) erstreckt.

15. Vorrichtung nach einem der Ansprüche 12 bis 14, ferner umfassend einen zweite Reinigungssäule (C62), wobei die erste Reinigungssäule (C61) mit einer vierten Rückführleitung und einer neunten Leitung verbunden ist, sich die vierte Rückführleitung von der Oberseite der Reinigungssäule (C61) zu einem Einlass der zweiten reaktiven Destillationssäule (C32) erstreckt, und sich die neunte Leitung von der Unterseite der ersten Reinigungssäule (C61) zu einem Einlass der zweiten Reinigungssäule (C62) erstreckt.

16. Vorrichtung nach Anspruch 15, wobei die zweite Reinigungssäule (C62) mit einer dritten Produktleitung und einer vierten Produktleitung verbunden ist, sich die dritte Produktleitung von der Oberseite der zweiten Reinigungssäule (C62) zum Bereitstellen von Diarylcarbonat erstreckt, und sich die vierte Produktleitung von der Unterseite der zweiten Reinigungssäule (C62) zum Bereitstellen eines Abfallstroms erstreckt.

17. Vorrichtung nach einem der Ansprüche 12 bis 16, wobei die zweite Rektifiziersäule (C41) einen Aufkocher umfasst und wobei die Wärme der Kondensation aus der ersten reaktiven Destillationssäule (C21) Wärme an den Aufkocher der zweiten Rektifiziersäule (C41) abgibt.

18. Vorrichtung nach einem der Ansprüche 12 bis 17, wobei das Dialkylcarbonat Dimethylcarbonat umfasst, das Diarylcarbonat Diphenylcarbonat umfasst, der aromatische Alkohol Phenol umfasst, das Alkylarylcarbonat Methylphenylcarbonat umfasst, der Alkylarylether Methylphenylether umfasst und der Alkylalkohol Methanol umfasst.

## Revendications

1. Procédé pour la production de carbonate de diaryle, comprenant :
l'introduction de réactifs comprenant du carbonate de dialkyle, un alcool aromatique, et un catalyseur de transestérification à une première colonne de distillation réactive (C21) pour produire du carbonate d'aryle d'alkyle et de l'alcool d'alkyle ;
la récupération à partir de la première colonne de distillation réactive (C21) d'un premier flux supérieur (flux 5) comprenant du carbonate de dialkyle et de l'alcool d'alkyle, un premier flux inférieur (flux 6) comprenant du carbonate d'aryle d'alkyle, de l'alcool aromatique, du carbonate de dialkyle, de l'éther d'alkyle d'aryle, et un catalyseur de transestérification, et un premier flux latéral (flux 13) comprenant du carbonate de dialkyle, de l'éther, et de l'alcool d'alkyle ;
l'introduction du premier flux inférieur (flux 6) dans une deuxième colonne de distillation réactive (C32) sans passer par une autre colonne de distillation réactive pour produire du carbonate de diaryle par la dismutation du carbonate d'aryle d'alkyle ;
la récupération à partir de la deuxième colonne de distillation réactive (C32) d'un deuxième flux inférieur (flux 15) comprenant du carbonate de diaryle et du carbonate d'aryle d'alkyle, et un deuxième flux supérieur (flux 16) comprenant de l'alcool aromatique, de l'éther d'aryle d'alkyle, et du carbonate de dialkyle, et recycler optionnellement le deuxième flux supérieur (flux 16) à la première colonne de distillation réactive (C21) ;
l'introduction du premier flux latéral (flux 13) à une première colonne de rectification (C81) ;
la récupération à partir de la première colonne de rectification (C81) d'un troisième flux inférieur (flux 12) comprenant de l'éther d'aryle d'alkyle et un troisième flux supérieur (flux 14) comprenant du carbonate de dialkyle et de l'alcool d'alkyle, et optionnellement le recyclage du troisième flux supérieur (flux 14) jusqu'à la première colonne de distillation réactive (C21) ;
l'introduction du premier flux supérieur (flux 5) dans une deuxième colonne de rectification (C41) ; et
la récupération à partir de la deuxième colonne de rectification (C41) d'un quatrième flux supérieur (flux 7) comprenant du carbonate de dialkyle / azéotrope d'alcool d'alkyle et un quatrième flux inférieur (flux 8) comprenant du carbonate de dialkyle, et optionnellement le recyclage du quatrième flux inférieur (flux 8) jusqu'à la première colonne de distillation réactive (C21) ;
l'introduction du deuxième flux inférieur (flux 15) à un ballon de flashing (V51) ;
la récupération à partir du ballon de flashing (V51) d'un cinquième flux inférieur (flux 10) comprenant un catalyseur et du carbonate de diaryle, et un cinquième flux supérieur (flux 9) comprenant du carbonate de diaryle, et du carbonate d'aryle d'alkyle et de l'alcool aromatique ;
l'introduction du cinquième flux inférieur (flux 10) à un évaporateur (C52) ; et
la récupération à partir de l'évaporateur (C52) d'un sixième flux supérieur (flux 11) comprenant du carbonate de diaryle et un sixième flux inférieur (flux 20) comprenant le catalyseur et le carbonate de diaryle, et le recyclage du catalyseur à une première colonne de distillation réactive (C21) ;
dans lequel le procédé ne comprend pas de colonnes réactives additionnelles autres que la première colonne de distillation réactive (C21) et la deuxième colonne de distillation réactive (C32).

2. Procédé selon la revendication 1, comprenant en outre :
l'introduction du sixième flux supérieur (flux 11) à une première colonne de distillation de purification (C61) ;
la récupération à partir de la première colonne de purification (C61) d'un septième flux inférieur (flux 23) comprenant du carbonate de diaryle et un septième flux supérieur (flux 17) comprenant du carbonate d'alkyle d'aryle qui n'a pas réagi et de l'alcool d'alkyle, et le recyclage du septième flux supérieur (flux 17) jusqu'à la deuxième colonne de distillation réactive (C32) ;
l'introduction du septième flux inférieur (flux 23) dans une deuxième colonne de purification (C62) ; et
la récupération à partir de la deuxième colonne de purification (C62) d'un huitième flux supérieur (flux 24) comprenant du carbonate de diaryle et un huitième flux inférieur (flux 25) comprenant des produits résiduels.

3. Procédé selon l'une quelconque des revendications 1-2, dans lequel la première colonne de distillation réactive (C21) est maintenue à une température de 120 °C à 270 °C, et une pression sur la partie supérieure de la première colonne de distillation réactive (C21) de 4 à 6 bars (400 à 600 kPa).

4. Procédé selon l'une quelconque des revendications 1-5, dans lequel la deuxième colonne de distillation réactive (C32) est maintenue à une température de 140°C à 240°C, et une pression sur la partie supérieure de la deuxième colonne de distillation réactive (C32) de 0,2 à 0,9 bars (20 à 90 kPa).

5. Procédé selon l'une quelconque des revendications 1-4, dans lequel la première colonne de rectification (C81) est maintenue à une température de 100°C à 190°C, et une pression sur la partie supérieure de la colonne (C81) de 1 à 3 bars (100 à 300 kPa).

6. Procédé selon l'une quelconque des revendications 1-5, dans lequel la deuxième colonne de rectification (C41) est maintenue de 50°C à 110°C, et une pression sur la partie supérieure de la colonne (C41) de 0,5 à 2 bars (50 à 200 kPa).

7. Procédé selon l'une quelconque des revendications 1-6, dans lequel la première colonne de purification (C61) est maintenue à une température de 60°C à 190°C, et une pression sur la partie supérieure de la colonne (C61) de 10 à 70 mbar (1 à 7 kPa).

8. Procédé selon l'une quelconque des revendications 1-7, dans lequel le catalyseur comprend un composé de titane, des composés d'organoétain, ou une combinaison comprenant au moins l'un des catalyseurs précédents.

9. Procédé selon l'une quelconque des revendications 1-8, dans lequel le catalyseur comprend du tétraphénoxyde de titane, de l'isopropylate de titane, du tétrachlorure de titane, ou une combinaison comprenant au moins l'un des catalyseurs précédents.

10. Procédé selon l'une quelconque des revendications 1-9, dans lequel le catalyseur comprend du tétraphénoxyde de titane.

11. Procédé selon l'une quelconque des revendications 1-10, dans lequel la première colonne de rectification (C41) comprend un rebouilleur, et dans lequel la chaleur de condensation de la première colonne de distillation réactive (C21) fournit de la chaleur à un rebouilleur d'une deuxième colonne de rectification (C41).

12. Procédé selon l'une quelconque des revendications 1-11, dans lequel le carbonate de dialkyle comprend du carbonate de diméthyle, le carbonate de diaryle comprend du carbonate de diphényle, l'alcool aromatique comprend du phénol, le carbonate d'aryle d'alkyle comprend du carbonate de phényle de méthyle, l'éther d'alkyle d'aryle comprend de l'éther de phényle de méthyle, et l'alcool d'alkyle comprend du méthanol.

13. Appareil pour la production continue de carbonate de diaryle par la réaction d'un carbonate de dialkyle et un alcool aromatique en présence d'un catalyseur de transestérification, comprenant :
une première colonne de distillation réactive (C21), une deuxième colonne de distillation réactive (C32), une première colonne de rectification (C81), une deuxième colonne de rectification (C41) et une pluralité de lignes pour transporter un réactif et des flux de produit ;
dans lequel la première colonne de distillation réactive (C21) est reliée à des lignes d'entrée pour l'introduction de réactifs, et à une première ligne de transfert, une deuxième ligne de transfert et une troisième ligne de transfert, la première ligne de transfert s'étend de la partie supérieure de la première colonne de distillation réactive (C21) à une entrée de la deuxième colonne de rectification (C41), la deuxième ligne de transfert s'étend de la partie inférieure de la première colonne de distillation réactive (C21) à une entrée de la deuxième colonne de distillation réactive (C32) sans passer par une autre colonne de distillation réactive, et la troisième ligne de transfert s'étend du côté de la première colonne de distillation réactive (C21) à une entrée de la première colonne de rectification (C81) ;
dans lequel la deuxième colonne de distillation réactive (C32) est optionnellement reliée à une première ligne de recyclage, la première ligne de recyclage s'étend de la partie supérieure de la deuxième colonne de distillation réactive (C32) à une entrée de la première colonne de distillation réactive (C21), et une quatrième ligne de transfert, la quatrième ligne de transfert s'étend de la partie inférieure de la deuxième colonne de distillation réactive (C32) pour fournir du carbonate de diaryle ;
dans lequel la première colonne de rectification (C81) est reliée à une cinquième ligne de transfert, la cinquième ligne de transfert s'étend de la partie supérieure de la première colonne de rectification (C81) à une entrée de la première colonne de distillation réactive (C21), et une première ligne de produit s'étend de la partie inférieure de la première colonne de rectification (C81) fournissant de l'éther d'aryle d'alkyle ; et
dans lequel la deuxième colonne de rectification (C41) est reliée à une deuxième ligne de produit pour fournir du carbonate de dialkyle/azéotrope d'alcool d'alkyle depuis la partie supérieure de la deuxième colonne de rectification (C41), et optionnellement une deuxième ligne de recyclage s'étend de la partie inférieure de la deuxième colonne de rectification (C41) à la partie inférieure de la première colonne de distillation réactive (C21)
comprenant en outre un ballon de flashing (V51), un évaporateur (C52), et une première colonne de purification (C61), dans lequel le ballon de flashing (V51) est relié à une sixième ligne et à une septième ligne, la sixième ligne s'étend de la partie supérieure du ballon de flashing (V51) à une entrée de la première colonne de purification (C61), et la septième ligne s'étend de la partie inférieure du ballon de flashing (V51) à une entrée de l'évaporateur (C52) ; et
dans lequel l'appareil ne comprend pas de colonnes réactives additionnelles autres que la première colonne de distillation réactive (C21) et la deuxième colonne de distillation réactive (C32).

14. Appareil selon la revendication 13, dans lequel l'évaporateur (C52) est relié à une huitième ligne et à une troisième ligne de recyclage, la huitième ligne s'étend de la partie supérieure de l'évaporateur (C52) à une entrée de la première colonne de purification (C61), et la troisième ligne de recyclage s'étend de la partie inférieure de l'évaporateur (C52) à une entrée de la première colonne de distillation réactive (C21).

15. Appareil selon l'une quelconque des revendications 12-14, comprenant en outre une deuxième colonne de purification (C62), dans lequel la première colonne de purification (C61) est reliée à une quatrième ligne de recyclage et à une neuvième ligne, la quatrième ligne de recyclage s'étend de la partie supérieure de la première colonne de purification (C61) à une entrée de la deuxième colonne de distillation réactive (C32), et la neuvième ligne s'étend de la partie inférieure de la première colonne de purification (C61) à une entrée de la deuxième colonne de purification (C62).

16. Appareil selon la revendication 15, dans lequel la deuxième colonne de purification (C62) est reliée à une troisième ligne de produit et à une quatrième ligne de produit, la troisième ligne de produit s'étend de la partie supérieure de la deuxième colonne de purification (C62) pour fournir du carbonate de diaryle, et la quatrième ligne de produit s'étend de la partie inférieure de la deuxième colonne de purification (C62) fournissant un flux de résidus.

17. Appareil selon l'une quelconque des revendications 12-16, dans lequel la deuxième colonne de rectification (C41) comprend un rebouilleur, et dans lequel la chaleur de condensation de la première colonne de distillation réactive (C21) fournit de la chaleur au rebouilleur de la deuxième colonne de rectification (C41).

18. Appareil selon l'une quelconque des revendications 12-17, dans lequel le carbonate de dialkyle comprend du carbonate de diméthyle, le carbonate de diaryle comprend du carbonate de diphényle, l'alcool aromatique comprend du phénol, le carbonate d'aryle d'alkyle comprend du carbonate de phényle de méthyle, l'éther d'aryle d'alkyle comprend l'éther de phényle de méthyle et l'alcool d'alkyle comprend du méthanol.
